# EUROPÄISCHE PATENTSCHRIFT

(11) **EP 2 377 460 B1**
(45) Veröffentlichungstag und Bekanntmachung des Hinweises auf die Patenterteilung: **17.04.2013**
(21) Anmeldenummer: 10160370.2
(22) Anmeldetag: 19.04.2010
(51) Int. Cl.: A61B 5/0205, G06F 19/00

(54) **System zur Früherkennung lebensbedrohlicher Zustände von Personen**
System for early recognition of people in hazardous states
Système de reconnaissance précoce d'états mettant la vie de personnes en danger

(43) Veröffentlichungstag der Anmeldung: 19.10.2011
(73) Patentinhaber: Schardey, Anne, 83703 Gmund (DE)
(72) Erfinder: Schardey, Anne, 83703 Gmund (DE)
(74) Vertreter: Winter, Brandl, Fürniss, Hübner, Röss, Kaiser, Polte - Partnerschaft

(56) Entgegenhaltungen:
- US-A1- 2007 238 995
- US-A1- 2008 146 895
- US-A1- 2008 167 567
- US-A1- 2008 287 753

## Beschreibung

Die Erfindung betrifft ein System zur Früherkennung lebensbedrohlicher Zustände von Personen.

Es sind Überwachungssysteme in verschiedenen Ausführungen bekannt, welche vorzugsweise in Krankenhäusern für die Patientenüberwachung zum Einsatz kommen. Dabei werden lediglich relevante Vitalparameter wie bspw. Puls, Herzfrequenz etc. überwacht, wobei bei Überschreitung der jeweiligen statischen Grenzwerte der Vitalparameter ein Alarm ausgelöst wird. Jedoch können im Verlauf nach einer Operation verschiedene Komplikationen wie zum Beispiel Schock, Herzinfarkt, Lungenembolie sowie Blutungen auftreten, die sich nicht durch eine triviale Grenzwertüberschreitung einzelner Vitalparameter bemerkbar machen.

Aus dem Dokument US 2008/0167567 A1 ist ein System zur Früherkennung lebensbedrohlicher Zustände gemäß dem Oberbegriff des Patentanspruchs 1 bekannt geworden. Hierbei geht es um die Vorhersage und die Vermeidung eines postoperativ auftretenden Vorhofflimmerns von Herzpatienten. Es werden Parameter erfasst, die die Aktivität des Herzens aus den EKG-Daten wiedergeben.

Aus dem Dokument US 2008/0287753 ist ein System und ein Verfahren zur Vorhersage und zur Erfassung eines Kreislaufschocks bekannt. Als Parameter zur Sicherstellung der Überwachungsfunktion dienen dabei Signale des EKG und einer Blutdruckmesseinheit.

Im Falle von Blutungen ist es wichtig, dass unerhebliche Hämatome von der inneren Nachblutung unterschieden werden, um die Gefahr des Verblutens bzw. der Kompression lebenswichtiger Organe zu erkennen. Das Problem der postoperativen Blutung wird vor dem Hintergrund, dass eine Tonsillektomie unter anderem der am häufigsten durchgeführte geplante Routineeingriff im operativen HNO-Bereich ist, wobei die häufigste Komplikation nach solch einer Operation Nachblutungen sind, besonders deutlich. Insbesondere Kinder, die wegen guter körperlichen Kompensation, geringer Kommunikationsfähigkeit sowie der Tendenz zur Panik und Agitation häufig in Folge einer unerkannten Nachblutung sterben, sind besonders gefährdet.

Es besteht ebenfalls die Gefahr der Kompression lebenswichtiger Organe, wie beispielsweise der Luftröhre, in Folge von Nachblutungen. In Deutschland kommt es pro Jahr in ca. 800 Fällen von Schilddrüsenoperationen zu Nachblutungen, wobei ca. 16 Patienten davon sterben und ungefähr weitere ebenso viele Patienten zu Apallikern werden. Da die oben genannten Gefahren mit den herkömmlichen Überwachungsarten nicht ausreichend sicher und frühzeitig erkannt werden können, besteht Handlungsbedarf bei der Weiterentwicklung aktueller Systeme.

Der Erfindung liegt deshalb die Aufgabe zugrunde, ein System zu schaffen, welches sich in besonderer Weise dazu eignet, lebensbedrohliche Zustände von Personen zu erkennen.

Diese Aufgabe wird hinsichtlich des Systems zur Früherkennung lebensbedrohlicher Zustände von Personen durch die Merkmale des Anspruchs 1 gelöst.

Das erfindungsmäßige System zur Früherkennung lebensbedrohlicher Zustände von Personen ist in der Lage, mehrere Vitalparameter einer zu überwachenden Person gleichzeitig zu verarbeiten. Diese Vitalparameter werden laufend von einer Detektoreinheit, die aus Messsonden und einer Recheneinheit, sowie einem Interface bestehen kann, einer Auswertelogik zugeführt, mit der die erfassten Parameter verschiedenen, vorbestimmten Zuständen zugeordnet werden und in Abhängigkeit der vorliegenden Parameterzustände eine Bewertung der Wahrscheinlichkeit des Vorliegens einer postoperativen Blutung vorgenommen wird. Die Erfinder haben erkannt, dass es dem automatisch arbeitenden System bei geeigneter Auswahl der zu überwachenden Vitalparameter und bei geeigneter Einordnung der gemessenen Parameter in die abzufragenden Zustände ohne weiteres gelingt, eine Wahrscheinlichkeit über den Zustand der zu überwachenden Person zu ermitteln, wobei eine klare Abgrenzung zwischen lebensbedrohlichen Zuständen einerseits und eher unkritischen Zuständen andererseits möglich ist. Wesentlicher Aspekt dabei ist die Erkenntnis, dass bei Verwendung einer Auswertelogik, die nicht digital, sondern mit Zwischenkriterien arbeitet, schon bei relativ wenigen zu überwachenden Parametern gelingt, lebensbedrohliche Zustände von gewöhnlichen Zuständen abzugrenzen. Über die Anzahl der Vitalparameter und/oder der Anzahl der Zustände, die den gemessenen Parametern zugeordnet werden, lässt sich die Arbeitsweise des Systems nicht nur beliebig verfeinern sondern auch den jeweiligen individuellen Reaktionsmustern der zu überwachenden Personen anpassen. Die Anzeigeeinrichtung, mit der die Bewertung zur Anzeige gebracht wird, ist vorteilhaft, weil sie einerseits dazu genutzt werden kann, der zu überwachenden Person einen Zustand zu signalisieren, der für sie kritisch ist, und sie gleichzeitig dazu genutzt werden kann, das Ergebnis der Auswertung in der Weise zu kommunizieren, beispielsweise an ein Krankenhaus oder an einen behandelnden Arzt, damit lebensrettenden Maßnahmen so schnell wie möglich anlaufen können. Es hat sich herausgestellt, dass es zum Beispiel mit der Überwachung von lediglich fünf Vitalparametern und einer Zuordnung dieser Vitalparameter zu lediglich fünf Zuständen bzw. Kategorien gelingt, die postoperative Blutung zuverlässig von unbedenklichen Gesundheitszuständen abzugrenzen, insbesondere dann, wenn diese Zustände individuell auf die zu überwachende Person abgestimmt werden. Ferner weist die Auswertelogik ein neuronales Netz auf, mit Hilfe dessen im Vorfeld trainierte Parametermuster erkannt werden.

Vorteilhafte Weiterbildungen sind Gegenstand der Unteransprüche.

Vorteilhafter Weise ist das Früherkennungssystem mit Hilfe der Auswertelogik zusätzlich in der Lage, auf der Grundlage der erfassten Vitalparameter und Parameterzustände, insbesondere unter Berücksichtigung deren zeitlichen Verlaufs, eine Anweisung des Inhalts zu erzeugen, wie mit der zu überwachenden Person umzugehen ist. Sie entspricht somit einer individuellen, den Gegebenheiten angepassten Handlungsempfehlung entweder für die zu überwachende Person selbst oder für Dritte, beispielsweise für einen behandelnden Arzt oder einen Ersthelfer, der die zu überwachende Person in einem kritischen Zustand antrifft. Da diese Handlungsempfehlung automatisch in Abhängigkeit der erfassten Parameterkonstellation generiert wird, kann sie auch dann verwertet werden, wenn die zu überwachende Person gar nicht mehr in der Lage ist, irgendwelche Auskünfte zu geben. Demzufolge kann in einem Notfall wertvolle Zeit zwischen der Diagnose und dem Start der Behandlung gewonnen werden.

Je nach Art der Anzeigeeinrichtung kann die Anweisung zumindest partiell zur Anzeige gebracht werden. Das ermöglicht in erster Linie der zu überwachenden Person den eigenen Zustand einzuschätzen und bei einem nicht lebensbedrohlichen Zustand sich nicht unnötig zu beunruhigen. Andernfalls kann die Anweisung bei einem lebensbedrohlichen Zustand unter anderem sein, dass unverzüglich ein Notarzt zu informieren sei. Parallel dazu können konkrete Anweisung angezeigt werden, wie mit der Person umzugehen ist. Somit kann beispielsweise der eintreffende Arzt sofort mit der richtigen Behandlung beginnen. Hieraus ergibt sich eine große Zeitersparnis, da dieser Arzt weder die zu überwachende Person untersuchen, noch zusätzliche Zeit für die Diagnosefindung aufwenden muss. Ferner, da das Früherkennungssystem die medizinische Historie der zu überwachende Person kennt, entspricht die Anweisung des Systems der, die ein Hausarzt anordnen würde, der mit der medizinischen Historie vertraut ist. Sollten für die Behandlung der zu überwachenden Person Spezialgeräte notwendig sein die nicht zu den standardmäßig mitgeführten Geräten der Notärzte gehören, kann durch eine Anweisung, die einen Hinweis auf diesen Umstand enthält, wiederum kostbare Zeit gewonnen werden.

Grundsätzlich kann als Detektoreinheit jegliche Einheit fungieren, die in Lage ist, die ausgewählten Vitalparameter zu erfassen. Wenn die Detektoreinheit Messsonden und ggf. eine Recheneinheit sowie ggf. ein Interface aufweist, wird das System in der Weise ausgebildet, dass es als körperbezogenes, kompaktes System am Körper der zu überwachenden Person getragen werden kann. Die Recheneinheit wandelt das Eingangssignal der Messsonden um, so dass es beispielsweise an einem externen Gerät über ein Interface sichtbar wird. Der Vorteil des Interfaces ist der, dass einerseits in einem Notfall die Vitalparameter der zu überwachenden Person für einen Fachmann, wie beispielsweise einem Arzt, schnell sichtbar gemacht werden können, und dass andererseits das System bei Routinekontrollen mit sehr geringem Aufwand ausgelesen werden kann.

Das erfindungsgemäße System überwacht sämtliche relevante Vitalparameter und ordnet diesen einen vorbestimmten Zustand zu. Mit Hilfe eines neuronalen Netzes gemäß Anspruch 4 ist die Auswertelogik des Systems ferner in der Lage, die große Anzahl von Parameterkonstellationen schnell zu handhaben und etwaige lebensbedrohliche Zustände verzögerungsfrei zu identifizieren. Des Weiteren können dem neuronalen Netz weitere, durch klinische Forschung bestimmte Parameterkonstellationen mittels Training vermittelt werden. Dadurch ist eine noch genauere Abgrenzung der Parameterkonstellationen zwischen einem unerheblichen und lebensbedrohlichen Zustand möglich.

Durch die Erfassung der Vitalparameter in vorbestimmten Zeitabständen (dt), die vorzugsweise an den gesundheitlichen Zustand des Patienten angepasst sind, kann das System besonders energiesparend eingesetzt werden, wodurch eine lange Betriebszeit erreicht wird.

Besondere Attraktivität gewinnt das System durch variable Zeitintervalle (dt) für die Erfassung der Vitalparameter. Dieses Merkmal erlaubt dem System die Abstände zwischen zwei Messpunkten in Abhängigkeit der Umstände zu variieren, so dass die Zeitintervalle während des Einsatzes des Systems an den aktuellen gesundheitlichen Zustand des Patienten angepasst werden können. So werden sie beispielsweise kürzer, wenn sich der Zustand verschlechtert, und länger, wenn sich der Zustand verbessert. Die Zeitintervalle (dt) können somit auch in unkritischen Phasen, wie beispielsweise beim Schlafen, länger werden, um wiederum eine energiesparende Arbeitsweise zu ermöglichen.

Durch die Weiterbildung des Anspruchs 6, in der die Auswertelogik auf einem Fuzzy-Logic-Ansatz, statt wie üblicherweise auf einem zweiwertigen, digitalen Ansatz, aufbaut, müssen die Vitalparameter nicht zwingend einem aus zwei möglichen, gegensätzlichen Zuständen zugeordnet werden, sondern auch beliebigen Zwischenwerten, wobei die Arbeitsgenauigkeit des Systems gesteigert und die Anzahl erkennbarer Zustände der zu überwachenden Person erhöht werden kann.

Je besser das System auf den Patienten angepasst ist, desto effektiver und genauer kann es arbeiten. Daher ist es von Vorteil, wenn das System mit individuellen medizinischen Daten der zu überwachenden Person trainiert ist. Auf diese Weise kann das System noch genauer zwischen einem kritischen, gesundheitlichen Zustand und unkritischen Zuständen, wie beispielsweise einer Ruhephase oder kurzen Stressphase, unterscheiden, insbesondere wenn sich die gemessenen Parameter von Person zu Person erheblich unterscheiden. Die Unterscheidung hat zur Folge, dass erheblich weniger Fehlalarme angezeigt bzw. gemeldet werden.

Durch die im Anspruch 8 dargestellte Weiterbildung kann das Fuzzy-Logic-Modul der Auswertelogik im Vorfeld individuell entsprechend dem gesundheitlichen Zustand der zu überwachenden Person geeicht werden. Dadurch fallen etwaige Anpassungsläufe bei Einsatzbeginn weg und der Patient wird von Anfang an bestmöglich überwacht.

Um das System im Einsatz laufend zu verbessern und an die individuellen Zustände des Patienten während dessen Einsatzdauer anzupassen, kann die Auswertelogik lernfähig ausgebildet sein. Das hat den Vorteil, dass sich das System während des Einsatzes an Gewohnheiten und Rhythmus des Patienten anpassen kann. Dadurch werden individuelle Vitalparameter und deren Verhältnis zueinander erfasst, womit wiederum genauere Aussagen bzgl. unerheblichen Zuständen, wie beispielsweise Schlafphasen oder kurzzeitige Anstrengungen, gegenüber lebensbedrohlichen Zuständen, wie beispielsweise postoperativen Blutungen, getroffen werden können.

Wenn eine Bewertung der Wahrscheinlichkeit des Vorliegens einer gesundheitlichen Anomalität gemäß dem Anspruch 10 zur Anzeige gebracht wird, kann das Ergebnis der Überwachung sehr leicht erfasst werden. Dabei hat die Anzeigeeinheit die Möglichkeit, verschiedene Stufen der Gesundheitsgefährdung beispielsweise optisch mittels verschieden farbiger Signalleuchten zu signalisieren. Das dient zur besseren Einschätzung des ausgelösten Alarms, da ein lebensbedrohlicher Zustand klar von lediglich leicht erhöhten Werten differenziert angezeigt werden kann. Dies verhindert wiederum eine unnötige Beunruhigung des Patienten, was letztendlich die weitere Verschlechterung des Gesundheitszustandes vermeidet. Zusätzlich können Fehlalarme und dadurch unnötig verursachte Kosten vermieden werden.

Durch die Weiterbildung des Anspruchs 11 kann zumindest ein ausgewähltes Ergebnis der Bewertung und/oder eine Anweisung, wie mit dem momentanen Zustand der zu überwachenden Person umzugehen ist, durch eine Sendeeinheit an eine Zentrale übermittelt werden. Auf diese Weise können entsprechenden Fachleute das Ergebnis der Bewertung unmittelbar analysieren und gegebenenfalls Handeln.

Um die räumliche bzw. geografische Position der zu überwachenden Person bestimmen zu können, kann die Sendeeinheit einen Positionsdetektor aufweisen. Dieser ist so ausgebildet, dass er vorzugsweise laufend die Position der zu überwachenden Person bestimmen kann.

Die Sendeeinheit kann derart ausgebildet sein, dass sie die bestimmte räumliche bzw. geografische Position der zu überwachenden Person an einer Zentrale übermittelt. Infolgedessen kann die zu überwachenden Person unmittelbar aufgefunden werden, falls ein lebensbedrohlicher Zustand eingetreten ist, selbst dann, wenn diese Person nicht selbstständig auf sich aufmerksam machen kann. Dadurch kann beispielsweise sowohl bei der Auffindung des Raumes eines stationären als auch bei nichtstationären Patienten wertvolle Zeit gewonnen werden.

Ferner kann das System des Anspruchs 1 mit einer Schnittstelle ausgestattet sein, die eine Verbindung zu einem Mobiltelefon erlaubt. Einerseits kann durch das Mobiltelefon auch in Örtlichkeiten eine Positionsbestimmung durchgeführt werden, in denen herkömmliche Systeme, wie beispielsweise GPS, nicht arbeiten, andererseits kann zum Übermitteln von Informationen auf das Mobiltelefon zurückgegriffen werden.

Besondere Praktikabilität und einfache Inbetriebnahme ergibt sich dann, wenn alle Komponenten des Systems zu einem Gerät zusammengefasst sind. Das hat zur Folge, dass dieses System als eine Einheit für jedermann käuflich erwerbbar und ohne Montageaufwand einsetzbar ist. Des Weiteren kann das System schnurlos ausgebildet sein, wodurch das Tragen am Körper, beispielsweise an Handgelenk, Gürtel oder ähnlichem, für den Anwender besonders erleichtert wird.

Nachstehend wird anhand schematischer Zeichnungen ein Ausführungsbeispiel der Erfindung näher erläutert. Es zeigen:
Fig. 1A bis 1C eine schematische Darstellung des Systems zur Früherkennung lebensbedrohlicher Zustände, welches von einer zu überwachenden Person getragen wird, ein Blockschaltbild der Auswertelogik sowie weitere Varianten von Anzeigen;
Fig. 2 Zeitverläufe von zwei Vitalparametern, deren Grenzwertüberschreitung zu gewissen Zeitpunkten jeweils einen Alarm auslöst;
Fig. 3A und 3B zwei mögliche Parameterkonstellationen einer zu überwachenden Person, Schlafen und Agitation; und
Fig. 4A und 4B zwei mögliche Parameterkonstellationen einer zu überwachenden Person, Blutung in eine Körperhöhle und Blutung in die OP-Loge.

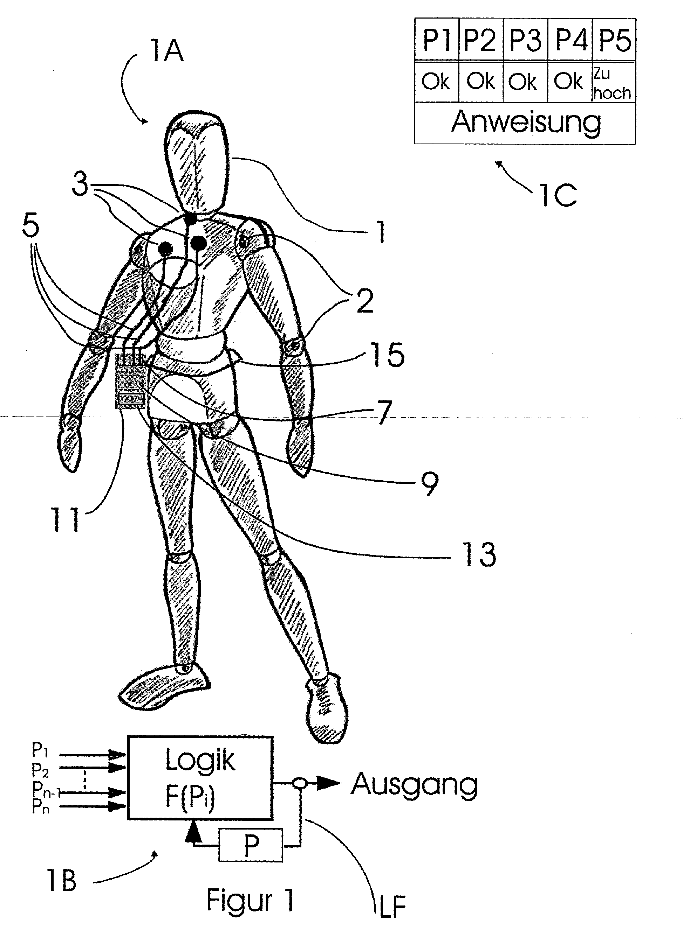

In Figur 1A ist mit dem Bezugszeichen 1 schematisch eine zu überwachende Person (nachfolgend Person genannt) bezeichnet. Dabei stellen die mit dem Bezugzeichen 2 gekennzeichneten Stellen Gelenke dar.

Zur Erfassung der Vitalparameter werden Messsonden 3, die durch runde Punkt dargestellt sind, an die Person angebracht. Je nach Vitalparameter werden die Messsonden 3 an unterschiedlichen Körperstellen der Person 1 angebracht. Die Datenübertragung erfolgt in dem gezeigten Ausführungsbeispiel über ein Kabel 5, wobei die Datenübertragung ebenso schnurlos via Infrarot oder Funk möglich ist. Die Kabel 5 sind an ihrem einen Ende mit der Messsonde 3, und an ihrem anderen Ende mit einem Interface 7 verbunden. Von dem Interface 7 aus gelangen die von den Messsonden 3 erfassten Vitalparameter an eine ECU 9, welche zusammen mit den eben genannten Elementen 3, 5 und 7 die Detektoreinheit bildet, welche wiederum komplett in einem Gehäuse 11 aufgenommen ist.

Bei der Detektoreinheit erfüllt das Interface 7 zwei Funktionen. Zum einen dient es als eine Art interne Schnittstelle der Detektoreinheit für die von den Messsonden 3 erfassten Vitalparametern. Zum anderen dient das Interface 7 als externe Schnittstelle, um externe Geräte, wie beispielsweise einen Computer oder Monitor, daran anschließen zu können.

Ferner enthält das Gehäuse 11 die ECU 9 der Detektoreinheit. Sie beinhaltet einen Taktgeber und führt somit sämtliche Rechenprozesse durch. Je nach Vitalparameter und Zustand der zu überwachenden Person können die Zeitintervalle zwischen zwei Messpunkten erheblich voneinander abweichen. Während beispielsweise Messungen der Herzfrequenz in deutlich kürzeren zeitlichen Abständen voneinander durchgeführt werden müssen um eine lebensbedrohliche Situation frühzeitig und schnell zu erkennen, sind Messungen bezüglich der Sauerstoffsättigung des Blutes auch in größeren Abständen ausreichend.

Weiterhin kann die ECU 9 so ausgestattet sein, dass sie auf einen Speicher zugreifen kann, in dem sämtliche relevante Anweisungen gespeichert sind, wie in Abhängigkeit von den momentan vorliegenden Vitalparametern mit der zu überwachenden Person umzugehen ist. Der Speicher kann dabei sowohl ein interner, als auch ein externer Speicher sein. Darin kann die ECU 9 sowohl die Anweisungen speichern, damit diese bei Bedarf ausgelesen werden können, als auch Verläufe bzw. Entwicklungen der Verläufe von Vitalparametern über längere Zeiträume speichern. Diese können beispielsweise bei Routinekontrollen über das Interface 7 ausgelesen und analysiert werden.

Der im Folgenden beschriebene Ablauf beschreibt das in Figur 1B dargestellte Blockschaltbild, wobei die Eingangsgrößen durch P₁ bis Pₙ, die Auswertelogik durch F(Pᵢ), der Rückführungspfad zur Lernfähigkeit durch die Bezeichnung LF und die Verarbeitung des Ergebnisses zur Anpassung der Auswertelogik für zukünftige Auswertungen durch die Bezeichnung P dargestellt ist. Je nach Behandlungsfall werden die erfassten Vitalparameter der ECU 9 zugeführt, welche dann, basierend auf einem Fuzzy-Logic-Ansatz, diese verschiedenen vorbestimmten Zuständen, wie z. B. sehr niedrig, niedrig, normal, hoch und sehr hoch, zuordnet.

Anschließend nimmt die Auswertelogik in Abhängigkeit der vorliegenden Parameterkonstellation eine Bewertung der Wahrscheinlichkeit des Vorliegens einer gesundheitlichen Anomalität vor. Dabei kann beispielsweise als Ergebnis die Wahrscheinlichkeit einer postoperativen Blutung ebenso wie die einer Thrombose, Embolie etc. bewertet ausgegeben werden. Nach Erhalt der Bewertung wird diese, falls es von der Logik als notwendig erachtet wird, mit einer Anzeigeeinrichtung 13 zur Anzeige gebracht.

Zusammen mit den Bewertungen können Anweisungen an der Anzeigeeinrichtung 13 ausgegeben werden, die je nach Anzeigeeinrichtung 13 partiell, durchlaufend oder mittels Symbolik ablesbar sind. Bei tendenziell kleineren Anzeigeeinrichtungen 13 können die Anweisungen nur partiell dargestellt werden, wohingegen eine größere Anzeigeeinrichtung 13 auch das Anzeigen ausführlicher Anweisungen erlaubt. Für einen Einsatzfall, bei dem nur eine besonders kleine Anzeigeeinrichtung 13 möglich ist, können Bewertungen, wie in Figur 1C dargestellt, mittels eindeutiger Symbole oder einer Kombination von Symbolen und Texten wiedergegeben werden. Dabei stehen in der oberen Zeile die erfassten Vitalparameter und in der mittleren Zeile jeweils deren Bewertung. Die untere Zeile kann leer sein oder den Hinweis anzeigen, dass alle Werte unkritisch sind, solange keine Gefahr für die zu überwachende Person besteht. Wird ein kritischer Zustand ermittelt, kann in diesem Textfeld eine Anweisung stehen, aufblinken oder ablaufen. Ferner können abwechselnd unterschiedliche Vitalparameter in den Spalten angezeigt werden, solange keine kritischen Werte erfasst werden.

Einzelne Anweisungen können beispielsweise folgendermaßen lauten:
- Achtung: Verdacht auf postoperative Blutung (Operationsdatum XX.XX.XXXX); Unverzüglich einen Arzt aufsuchen!
- Sauerstoffkonzentration fällt übermäßig ab. Konsultieren Sie Ihren Arzt unter der Telefonnummer: ... ... ...;
- Achtung: Erhöhter Puls. Bitte Ruhephase einleiten!

Ferner wird das Ergebnis der Auswertelogik, da Vitalparameter je nach momentaner Aktivität der Person erheblich voneinander abweichen können, dieser über den Verarbeitungsschritt P wieder zugeführt. Dadurch lernt das System mögliche unkritische Zustände, wie beispielsweise Ruhezustände, von kritischen Zuständen besser zu unterscheiden.

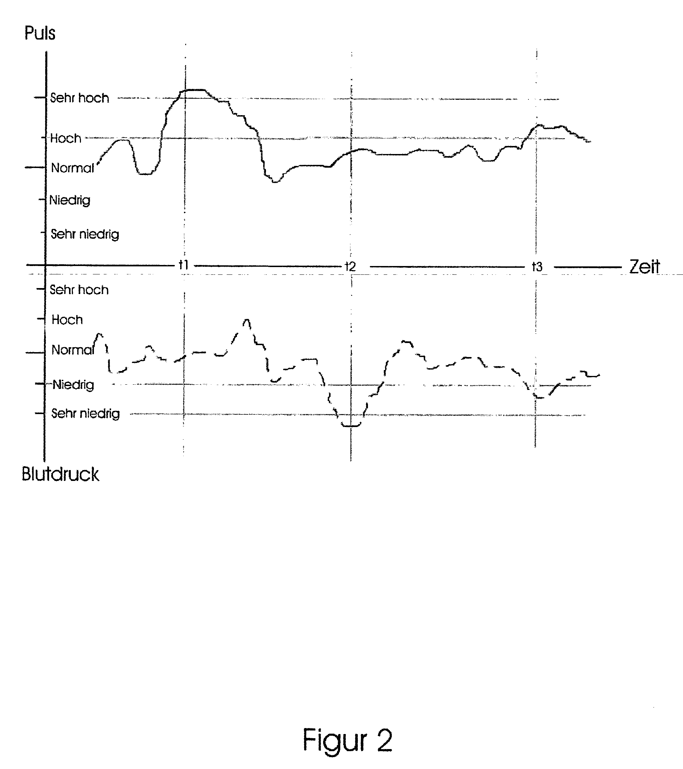

Beispielhafte Zeitverläufe von zwei Vitalparametern sind in den Figuren 2A und 2B gezeigt. Die Abszisse entspricht der Zeitachse, auf der beispielhaft drei verschiedene Zeitpunkte (t1, t2, t3) eingezeichnet sind. Auf der Ordinate sind oben die Zustände für den Puls (durchgezogene Linie) und unten die Zustände für den Blutdruck (gestrichelte Linie) angetragen. Die beiden Zeitpunkte t1 und t2 entsprechen jeweils einer Grenzüberschreitung über einen maximal zugelassenen Wert für den jeweiligen Vitalparameter. Dabei überschreitet beim Zeitpunkt t1 der Puls den dafür vorbestimmten oberen Grenzwert und wird dem Zustand sehr hoch zugeordnet. Zum Zeitpunkt t2 unterschreitet der Blutdruck den dafür vorbestimmten unteren Grenzwert und wird dem Zustand sehr niedrig zugeordnet. In beiden Fällen löst die Auswertelogik einen Alarm aus, um auf einen lebensbedrohlichen Zustand der Person 1 aufmerksam zu machen.

Dahingegen liegt zum Zeitpunkt t3 ein anderer Fall vor. Hier wurde nicht, wie in den beiden vorangehenden Fällen, ein Grenzwert überschritten, sondern von der Fuzzy-Logic erkannt, dass ungewöhnlich hohe Werte für zwei Vitalparameter aufgetreten sind. Basierend auf der Parameterkonstellation wird von einem lernfähigen neuronalen Netz der Auswertelogik eine Wahrscheinlichkeit für eine Diagnose berechnet und bewertet.

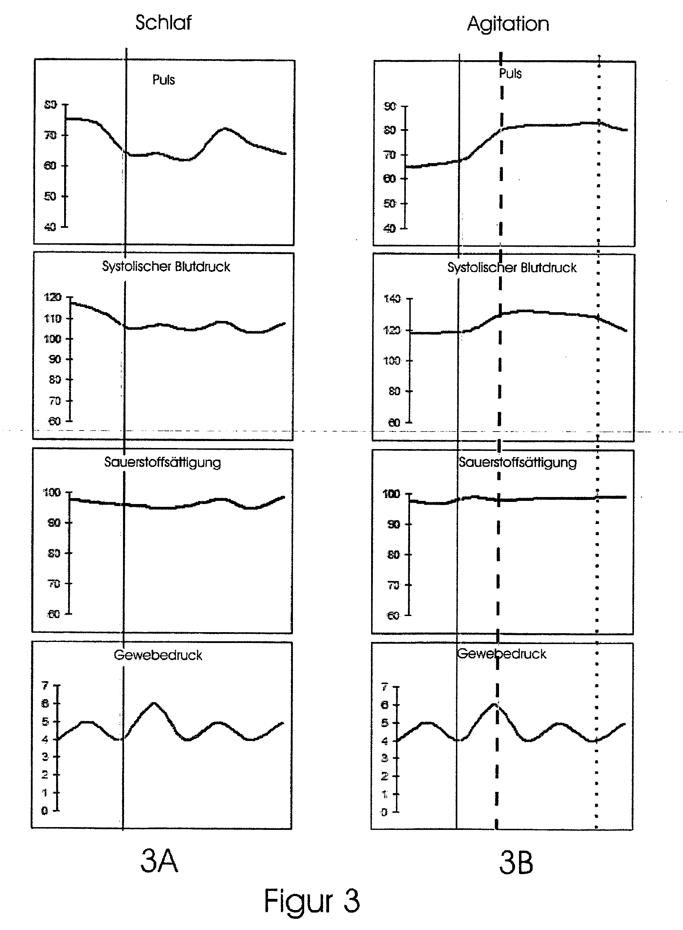

Die Figuren 3A (linke Spalte) und 3B (rechte Spalte) zeigen beispielhaft die Konstellation von vier Parametern für die Fälle Schlaf und Agitation. Die linken vier Felder von Figur 3A zeigen von oben nach unten die Verläufe für Puls, systolischen Blutdruck, Sauerstoffsättigung und Gewebedruck. Dabei sind die genannten Werte jeweils auf der Ordinate aufgezeichnet, während die Abszisse dem zeitlichen Verlauf entspricht. Der Eintritt eines Ereignisses ist durch eine durchgezogene senkrechte Linie dargestellt.

Ab dem Eintritt der Schlafphase sind Veränderungen im Verlauf der Vitalparameter zum einen am deutlich abnehmenden Puls, zum anderen am abnehmenden systolischen Blutdruck zu erkennen. Die beiden anderen Vitalparameter Sauerstoffsättigung und Gewebedruck bleiben nahezu unverändert. Dieser Parameterkonstellation deutet das neuronale Netz eindeutig als Schlafphase und veranlasst demzufolge keinen Alarm an der Anzeigeeinrichtung.

Die vier Felder von Figur 3B entsprechen denen von Figur 3A, jedoch ist hier die Parameterkonstellation der Agitation dargestellt. Der Eintritt eines Ereignisses ist wie vorher durch eine durchzogene senkrechte Linie dargestellt. Die gestrichelte senkrechte Linie stellt einen ersten Alarm dar. Das heißt, dass eine kritische Parameterkonstellation erkannt wurde und eine Diagnostik erforderlich ist, um einen lebensbedrohlichen Zustand zu vermeiden. In dem angeführten Beispiel ist der Puls deutlich gestiegen, während quasi zeitgleich der systolische Blutdruck ebenfalls gestiegen ist. In den beiden unteren Verläufen ist nahezu keine Änderung eingetreten. Demzufolge liegt bei der Person eine Agitation vor. Betrachtet man die Verläufe zu weiter vorangeschrittener Zeit (senkrechte strichpunktierte Linie), so weisen sowohl der Puls, als auch der systolische Blutdruck eine fallende Tendenz auf, was in der Gesamtheit der betrachteten Vitalparameter auf einer Normalisierung des Zustandes der Person hindeutet. Dies wird durch eine senkrechte strichpunktierte Linie dargestellt, welche aussagt, dass das Früherkennungssystem einwandfrei arbeitet und sich die Person in einem unkritischen Zustand befindet.

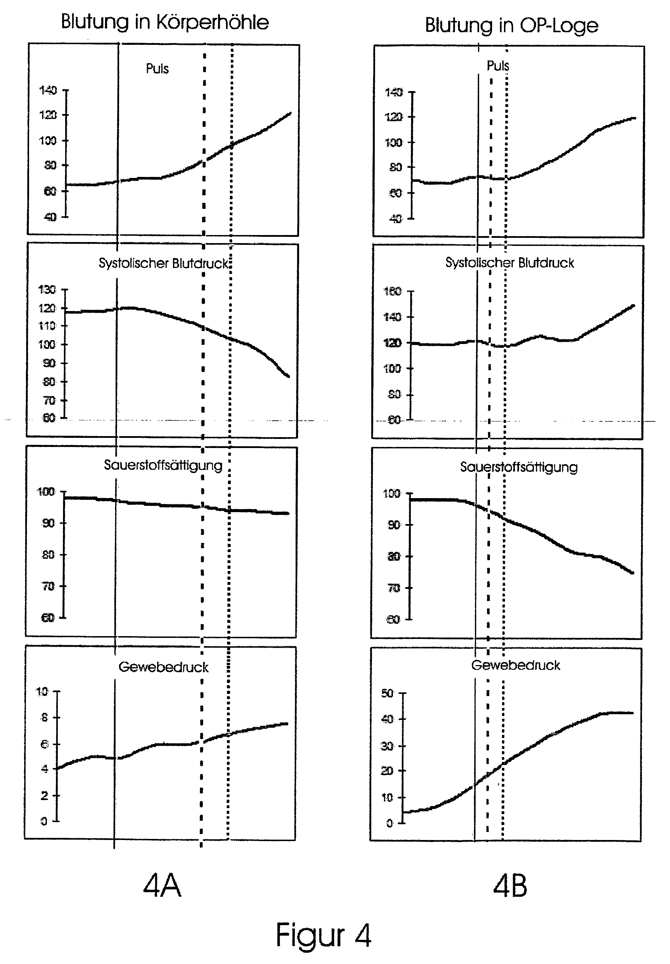

Zwei weitere mögliche Parameterkonstellationen sind in den Figuren 4A (linke Spalte) und 4B (rechte Spalte) dargestellt. In Figur 4A sind nach Eintritt eines Ereignisses (dargestellt durch die senkrechte durchgezogene Linie) Änderungen bei drei Vitalparametern zu erkennen. Während der Puls erst schwächer, dann jedoch immer stärker steigt, fällt der systolische Blutdruck. Parallel dazu steigt der Gewebedruck, während die Sauerstoffsättigung nahezu unverändert verläuft. In Kombination dieser drei Vitalparameter erfolgt ab der senkrechten gestrichelten Linie der erste Alarm, der auf die Notwendigkeit einer Diagnostik hinweist.

Bei der eben beschriebenen Parameterkonstellation würde die Anzeigeeinrichtung den Hinweis auf eine Blutung in eine Körperhöhle anzeigen. Da sich keiner der Verläufe wieder normalisiert sondern alle verschlechtern, wird ab der senkrechten strichpunktierten Linie ein Notfallalarm ausgelöst. Dieser weist darauf hin, dass sofort gehandelt werden muss, da sich die Person in einem lebensbedrohlichen Zustand befindet.

Figur 4B stellt eine weitere Parameterkonstellation dar, in der alle vier betrachteten Vitalparameter einen veränderten Verlauf nach dem Eintritt eines Ereignisses (dargestellt durch die senkrechte durchgezogene Linie) zeigen. Unmittelbar nach dem Ereignis beginnt zum einen die Sauerstoffsättigung zu fallen, zum anderen der Gewebedruck zu steigen. Da die Werte dieser beiden Parameter schnell kritische Werte erreichen (dargestellt durch die senkrechte gestrichelte und nachfolgend senkrechte strichpunktierte Linie), werden analog der beiden genannten Linien der erste Alarm und anschließend der Notfallalarm für einen lebensbedrohlichen Zustand ausgelöst.

Selbstverständlich sind Abweichungen von den zuvor beschriebenen Ausführungsbeispielen mögliche, ohne den Grundgedanken der Erfindung zu verlassen.

So ist es gleichermaßen möglich, die Messsonden an jeder anderen Stelle des Körpers anzubringen, der sich zur Erfassung der jeweiligen Vitalparameter eignet. Ebenso unterliegen die Messsonden keiner Beschränkung bzgl. ihre Form bzw. Größe.

Es ist selbstverständlich auch möglich, die erfassten Signale der Messsonden auf andere Weise an das System zu übermitteln, wie beispielsweise schnurlos mittels einer Infrarot- bzw. Funkverbindung.

Ferner ist es denkbar, dass das System nicht zwingend an der Hüfte der Person getragen wird, es kann auch um den Hals hängend oder am Arm oder Bein getragen werden.

Verschiedene Modifikationen der Anzeigeeinrichtung sind denkbar, mit denen die Stufen der Gesundheitsgefährdung zur Anzeige gebracht werden können. Eine Art Ampelanzeige mit den drei Farben grün, gelb und rot ist genauso denkbar wie eine Anzeige, die Balken oder andere Elemente verschiedener Größe anzeigt.

Abgesehen davon, dass die zu überwachende Person das System bei sich trägt, kann es sich ebenso an einem anderen Ort befinden, wie beispielsweise beim behandelnden Arzt, in einem Krankenhaus oder einer eigens dafür eingerichteten Überwachungsstelle.

Auch Grenzwertüberschreitungen anderer Vitalparameter als Puls und Herzfrequenz führen zum Auslösen des Alarms, d.h. Herzfrequenz, Atemfrequenz, Sauerstoffgehalt im Blut, Gewebedruck, etc. Ebenso kann ein verändertes EKG, welches auf eine akute Herzerkrankung, wie zum Beispiel einen Herzinfarkt hindeutet, zum Auslösen des Alarms führen.

Selbstverständlich können auch andere als nur die gezeigten Parameterkonstellationen zum Auslösen eines Alarms führen. Sämtliche bekannten und für die zu überwachende Person relevanten Parameterkonstellationen können dem System trainiert und demzufolge vom System erkannt werden.

Abgesehen von den oben beschriebenen Anwendungsfällen kann das System der vorliegenden Erfindung auch im Wellnessbereich zur Anwendung kommen. Dabei könnte das Gerät auch älteren Freizeit- und Extremsportlern die Sicherheit einer gesundheitlichen Überwachung ermöglichen.

Die Erfindung schafft somit ein System zur Früherkennung lebensbedrohlicher Zustände von Personen, bei denen solche Gefahren beispielsweise aufgrund einer vorangehenden Operation bestehen. Das System erfasst in Abhängigkeit der zu überwachenden Person mehrere Vitalparameter über eine Detektoreinheit, die anschließend von einer Auswertelogik, vorzugsweise unter Verwendung eines neuronalen Netzes, ausgewertet werden. Dabei werden zum einen Grenzwertüberschreitungen einzelner Parameter, zum anderen Parameterkonstellationen, die einen gesundheitlich kritischen Zustand der Person darstellen, mit einer Anzeigeeinrichtung je nach Gefährdung in unterschiedlichen Formen zur Anzeige gebracht. Sie wird bestimmt, in dem zunächst die Wahrscheinlichkeit für das Vorliegen einer gesundheitlichen Anomalität bestimmt und anschließend bewertet wird.

## Patentansprüche

1. System zur Früherkennung lebensbedrohlicher Zustände von Personen, mit einer Detektoreinheit zur Erfassung mehrerer Vitalparameter, wobei die Detektoreinheit vorzugsweise Messsonden (3) und ggf. eine Recheneinheit (9) sowie ggf. ein Interface (7) aufweist, wobei die Detektoreinheit die Parameter
a) Blutdruck RR,
b) Herzfrequenz und/oder Atmungsfrequenz,
c) Sauerstoffgehalt im Blut und
d) Gewebedruck
erfasst; mit
einer Auswertelogik, mit der die gemessenen und von der Detektoreinheit laufend zugeführte Parameter verschiedenen Zuständen, wie z.B. sehr niedrig, niedrig, normal, hoch und sehr hoch, zugeordnet werden, und welche in Abhängigkeit der vorliegenden Parameterzustände eine Bewertung der Wahrscheinlichkeit des Vorliegens einer postoperativen Blutung, vornimmt, und mit
einer Anzeigeeinrichtung (13), mit der die Bewertung zur Anzeige gebracht wird, wobei die Anzeigeeinrichtung (13) die Wahrscheinlichkeit einer postoperativen Blutung zur Anzeige bringt.

2. System nach Anspruch 1, **dadurch gekennzeichnet, dass** die Auswertelogik auf der Grundlage der erfassten Parameter und Parameterzustände, insbesondere unter Berücksichtigung deren zeitlichen Verlaufs, eine Anweisung erzeugt, wie mit dem momentanen Zustand der zu überwachenden Person umzugehen ist.

3. System nach Anspruch 2, **dadurch gekennzeichnet, dass** die Anweisung mittels der Anzeigeeinrichtung (13) zumindest partiell zur Anzeige gebracht werden kann.

4. System nach einem der Ansprüche 1 bis 3, **dadurch gekennzeichnet, dass** die Auswertelogik ein neuronales Netz aufweist.

5. System nach einem der Ansprüche 1 bis 4, **dadurch gekennzeichnet, dass** die Detektoreinheit zumindest einen der Vitalparameter in vorbestimmten Zeitabständen (dt) erfasst, die vorzugsweise variabel sind.

6. System nach einem der Ansprüche 1 bis 5, **dadurch gekennzeichnet, dass** die Auswertelogik auf einem Fuzzy-Logic-Ansatz aufgebaut ist.

7. System nach einem der Ansprüche 4 bis 6, **dadurch gekennzeichnet, dass** das neuronale Netz unter Heranziehung der individuellen medizinischen Daten der zu überwachenden Person trainierbar ist.

8. System nach Anspruch 6 oder 7, **dadurch gekennzeichnet, dass** ein Fuzzy-Logic-Modul der Auswertelogik individuell entsprechend dem gesundheitlichen Zustand der zu überwachenden Person geeicht wird.

9. System nach einem der Ansprüche 1 bis 8, **dadurch gekennzeichnet, dass** die Auswertelogik lernfähig ist.

10. System nach einem der Ansprüche 1 bis 9, **dadurch gekennzeichnet, dass** mit der Anzeigeeinheit verschiedene Stufen der Gesundheitsgefährdung zur Anzeige bringbar sind, z.B. optisch mittels verschieden farbiger Signalleuchten.

11. System nach einem der Ansprüche 1 bis 10, **gekennzeichnet durch** eine Sendeeinheit, mit der zumindest ein ausgewähltes Ergebnis der Bewertung und/oder eine Anweisung, wie mit dem momentanen Zustand der zu überwachenden Person umzugehen ist, an eine Zentrale übermittelbar ist.

12. System nach Anspruch 11, **gekennzeichnet durch** einen Positionsdetektor, mit dem die räumliche bzw. geografische Position der zu überwachenden Person vorzugsweise laufend bestimmbar ist.

13. System nach Anspruch 12, **dadurch gekennzeichnet, dass** die räumliche bzw. geografische Position der zu überwachenden Person mittels der Sendeeinheit an eine Zentrale übermittelbar ist.

14. System nach einem der Ansprüche 1 bis 13, **gekennzeichnet durch** eine Schnittstelle zu einem Mobiltelefon.

15. System nach einem der Ansprüche 1 bis 14, **dadurch gekennzeichnet, dass** sämtliche Elemente des Systems zu einer Einheit zusammengefasst sind.

## Claims

1. A system for the early detection of life-threatening conditions in human beings, with
a detector unit to record several vital parameters, whereby the detector unit preferably has probes (3) and possibly a calculation unit (9) and possibly an interface (7), whereby the detector unit records the parameters
a) RR blood pressure
b) heart rate and/or respiratory rate
c) oxygen content of the blood and
d) tissue pressure; with
evaluation logic with which the measured parameters, supplied continuously by the detector unit, are assigned to various conditions, for example very low, low, normal, high and very high, and which, depending on the existing parameter conditions, assesses the probability of the existence of post-operative bleeding, and with
a display device (13) with which the assessment is displayed, whereby the display device (13) displays the probability of post-operative bleeding.

2. A system in accordance with claim 1, **characterised in that** the evaluation logic generates an instruction, on the basis of the parameters and parameter conditions recorded, in particular in consideration of their change over time, for how to deal with the current condition of the person to be monitored.

3. A system in accordance with claim 2, **characterised in that** the instruction can be displayed at least partially by means of the display device (13).

4. A system in accordance with one of claims 1 to 3, **characterised in that** the evaluation logic has a neuronal network.

5. A system in accordance with one of claims 1 to 4, **characterised in that** the detector unit records at least one of the vital parameters at predetermined intervals of time (dt), which are preferably variable.

6. A system in accordance with one of claims 1 to 5, **characterised in that** the evaluation logic is based on a fuzzy logic approach.

7. A system in accordance with one of claims 4 to 6, **characterised in that** the neuronal network can be trained using the individual medical data items relating to the person to be monitored.

8. A system in accordance with claim 6 or 7, **characterised in that** a fuzzy logic module of the evaluation logic is calibrated individually according to the condition of health of the person to be monitored.

9. A system in accordance with one of claims 1 to 8, **characterised in that** the evaluation logic is capable of learning.

10. A system in accordance with one of claims 1 to 9, **characterised in that** different stages of danger to health can be displayed with the display unit, for example optically by means of signal lamps in different colours.

11. A system in accordance with one of claims 1 to 10, **characterised by** a transmission unit with which at least a selected result of the assessment and/or an instruction for how to deal with the current condition of the person to be monitored can be transmitted to a central unit.

12. A system in accordance with claim 11, **characterised by** a position detector with which the spatial or geographical position of the person to be monitored can be determined, preferably continuously.

13. A system in accordance with claim 12, **characterised in that** the spatial or geographical position of the person to be monitored can be transmitted to a central unit by means of the transmission unit.

14. A system in accordance with one of claims 1 to 13, **characterised by** an interface with a mobile phone.

15. A system in accordance with one of claims 1 to 14, **characterised in that** all elements of the system are combined as one unit.

## Revendications

1. Système de reconnaissance précoce d'états menaçant le pronostic vital de personnes, comportant une unité de détection pour la saisie de plusieurs paramètres vitaux, l'unité de détection présentant de préférence des sondes de mesure (3) et éventuellement, une unité de calcul (9) et éventuellement une interface (7) ; l'unité de détection captant les paramètres de
a) pression sanguine RR,
b) fréquence cardiaque et/ou la fréquence respiratoire,
c) teneur en oxygène dans le sang et
d) pression tissulaire
avec une logique d'évaluation avec laquelle les paramètres mesurés et acheminés en continu par l'unité de détection sont affectés à différents états, tels que par exemple, très faible, faible, normal, élevé et très élevé et qui entreprend en fonction des états de paramètres disponibles, une évaluation de la probabilité de la présence d'une hémorragie postopératoire et avec
un dispositif d'affichage (13) avec lequel l'évaluation est affichée,
le dispositif d'affichage (13) affichant une probabilité d'une hémorragie postopératoire.

2. Système selon la revendication 1, **caractérisé en ce que** la logique d'évaluation génère sur la base des paramètres captés et des états de paramètres, en particulier, en tenant compte de l'évolution temporelle, une instruction de traitement de l'état momentané de la personne à surveiller.

3. Système selon la revendication 2, **caractérisé en ce que** l'instruction peut être affichée au moins partiellement au moyen du dispositif d'affichage (13).

4. Système selon l'une des revendications 1 à 3, **caractérisé en ce que** la logique d'évaluation présente un réseau neuronal.

5. Système selon l'une des revendications 1 à 4, **caractérisé en ce que** l'unité de détection capte au moins l'un des paramètres vitaux à intervalles temporels prédéterminés (dt) qui sont de préférence variables.

6. Système selon l'une des revendications 1 à 5, **caractérisé en ce que** la logique d'évaluation est montée sur une installation de logique floue.

7. Système selon l'une des revendications 4 à 6, **caractérisé en ce que** le réseau neuronal peut être initié en référence aux données médicales individuelles de la personne à surveiller.

8. Système selon la revendication 6 ou 7, **caractérisé en ce qu'**un module de logique floue de la logique d'évaluation est étalonné individuellement de façon correspondant à l'état de santé de la personne à surveiller.

9. Système selon l'une des revendications 1 à 8, **caractérisé en ce que** la logique d'évaluation est capable de retenir.

10. Système selon l'une des revendications 1 à 9, **caractérisé en ce qu'**avec l'unité d'affichage, différentes étapes de la menace pour la santé peuvent être affichées, par exemple, optiquement au moyen de signaux lumineux de différentes couleurs.

11. Système selon l'une des revendications 1 à 10, **caractérisé par** une unité d'émission avec laquelle au moins un résultat d'évaluation choisi et/ou une instruction sur la façon de traiter l'état momentané de la personne à surveiller, peut être transmis(e) à une centrale.

12. Système selon la revendication 11, **caractérisé par** un détecteur de position avec lequel la position spatiale ou géographique de la personne à surveiller peut être déterminée, de préférence en continu.

13. Système selon la revendication 12, **caractérisé en ce que** la position spatiale ou géographique de la personne à surveiller peut être transmise à une centrale au moyen de l'unité d'émission.

14. Système selon l'une des revendications 1 à 13, **caractérisé par** une interface avec un téléphone mobile.

15. Système selon l'une des revendications 1 à 14, **caractérisé en ce que** tous les éléments du système sont regroupés en une unité.
